(19) Europäisches Patentamt
European Patent Office
Office européen des brevets

(11) **EP 3 466 389 B1**

(12) **EUROPEAN PATENT SPECIFICATION**

(45) Date of publication and mention
of the grant of the patent:
**20.05.2020 Bulletin 2020/21**

(51) Int Cl.:
*A61F 13/537* (2006.01)     *A61L 15/22* (2006.01)
*A61L 15/26* (2006.01)      *D04H 1/435* (2012.01)
*D04H 1/64* (2012.01)

(21) Application number: **17195230.2**

(22) Date of filing: **06.10.2017**

(54) **ABSORBENT ARTICLE COMPRISING A CARDED RESIN-BONDED NONWOVEN WEB WITH FIBERS COMPRISING ANTIMONY-FREE POLYETHYLENE TEREPHTHALATE**

ABSORBIERENDER ARTIKEL MIT EINER KARDIERTEN HARZVERKLEBTEN VLIESBAHN MIT FASERN MIT ANTIMONFREIEM POLYETHYLENTEREPHTHALAT

ARTICLE ABSORBANT COMPRENANT UNE BANDE NON TISSÉE LIÉE PAR RÉSINE CARDÉE AVEC DES FIBRES CONTENANT DU POLYÉTHYLÈNE TÉRÉPHTALATE SANS ANTIMOINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.04.2019 Bulletin 2019/15**

(73) Proprietor: **The Procter & Gamble Company Cincinnati, OH 45202 (US)**

(72) Inventors:
• **Saevecke, Dirk**
  **65824 Schwalbach am Taunus (DE)**
• **Virtanen, Otto**
  **65824 Schwalbach am Taunus (DE)**

(74) Representative: **P&G Patent Germany Procter & Gamble Service GmbH Sulzbacher Straße 40 65824 Schwalbach am Taunus (DE)**

(56) References cited:
**EP-A1- 1 584 723     EP-A1- 3 216 433
WO-A1-00/12792        WO-A1-91/18036**

**Description**

FIELD OF THE INVENTION

The invention provides an absorbent article for personal hygiene such as a diaper or pant (for babies, toddlers or adults), a training pant, or a feminine hygiene sanitary napkin. The absorbent article comprises a nonwoven web. The nonwoven web comprises staple fibers and a latex binder. The staple fibers comprise PET having less than 150 ppm of antimony.

BACKGROUND OF THE INVENTION

Polyethylene terephthalate (PET) is a well known and widely used material. The majority of the world's production of PET is for synthetic fibers (in excess of 60%), with bottle production accounting for about 30% of global demand. In the context of textile applications, PET is typically referred to by its common name, polyester.

PET is also often used in absorbent articles: Many absorbent articles, such as diapers, pants and feminine hygiene articles, comprise one or more nonwoven webs which comprise polyethylene terephthalate (PET).

PET is industrially produced by esterification or transesterification of terephthalic acid or dimethyl terephthalate and ethylene glycol to produce bis(2-hydroxyethyl) terephthalate which is then subjected to polycondensation at high temperatures in vacuo in the presence of a catalyst. As a conventional polyester polymerization catalyst used in polycondensation of polyester, antimony trioxide.

As a consequence of the use of an antimony compound as catalyst, traces of the antimony can be found in the PET resin and thus, also in the nonwoven web comprised by the absorbent article. Trace amounts of antimony in PET are typically in the range of 200 to 300 ppm.

Antimony is reported to have a negative impact on the environment and carcinogenic potential. Though the typical amounts of antimony in the PET resin are extremely low and not considered to be critical, increased attention on this chemical compound has been raised by consumers.

PET resin made by a process, which uses a catalyst other than antimony compounds, is known in the art, for example in CA02420958 assigned to Toyo Boseki Kabushiki Kaisha, JP; EP1316585B1 assigned to Invista Technologies, CH; EP1491572A1 assigned to Toray Industries, Inc, JP; EP1153953B1 and EP1327648A1, both assigned to Toyo Boseki Kabushiki Kaisha, JP. For example, a known approach to PET resin processing technology is to implement a titanium-containing polycondensation catalyst as a replacement for the conventional antimony-containing polycondensation catalyst. However, such titanium-containing polycondensation catalyst typically gives a yellowish color to the resultant PET resin, rendering the PET polyester fiber manufactured therefrom less commercially desired due to its yellowish look.

To reduce the yellowish look, it has been suggested to add a phosphorus stabilizer during the PET resin process in order to reduce the yellowish look of the PET resin caused by the titanium-containing polycondensation catalyst. For instance, U.S. Patent Application Publication US 2006/0014920A1 assigned to Teijin Fibers discloses a mixture-based catalyst mixed by tetrabutyltitanate (TBT), product of reaction of TBT and trimellitic anhydride, and triethyl phosphonoacetate (TEPA).

Though antimony free PET has been disclosed in the prior art, to date it has not found wide use in the industry, as most commercially available antimony free PET resins still have a yellowish color.

In absorbent articles, the use of antimony free PET has not been suggested so far. One of the reasons is supposed to be related to the yellowish color, which consumers perceive as low quality. This appears to be specifically critical as absorbent articles get into direct contact with the delicate skin, especially the skin of babies and toddlers.

EP1584723A1discloses polyester fiber structures which are said to have good color tones (low b* values) and being excellent in moldability.

EP3216433A1 refers to a carded nonwoven fibrous web. The web comprises at least 50%, by weight of the fibrous web, of staple fibers and at least 10%, by weight of the fibrous web, of non-fibrous latex binder, wherein, the staple fibers are autogenously bonded to each other and are bonded to each other by the latex binder.

WO91/18036 is concerned with polyesters, fibers and films, nonwovens from the fibers and disposable products of the polyesters such as diapers from the nonwovens. The products are said to be degradable under the conditions typically existing in waste composting processes, have low ingredient costs and yet provide strength and toughness properties adequate for end uses such as in disposable diapers.

SUMMARY OF THE INVENTION

Absorbent article comprising a topsheet forming a wearer-facing surface of the absorbent article, a backsheet forming a garment-facing surface of the absorbent article and an absorbent core interposed between the topsheet and the backsheet. The absorbent article comprises a nonwoven web, which comprises at least 50%, by weight of the nonwoven web, of staple fibers and at least 10%, by weight of the nonwoven web, of a latex binder. The staple fibers comprise at least 10 % by weight of the staple fibers, of polyethylene terephthalate (PET), the PET comprising less than 150 ppm of antimony. The staple fibers have an a* value of unequal zero and a b* value of unequal zero.

The nonwoven web may comprise at least 30%, or at least 50%, or at least 70%, or 100%, by weight of the staple fibers, of PET with less than 150 ppm of antimony. All staple fibers of the nonwoven web may comprise or consist of PET with less than 150 ppm of antimony.

The PET comprised by the staple fibers of the nonwoven web may have less than 100 ppm, less than 75 ppm, less than 50 ppm, less than 10 ppm of antimony, or may be completely antimony-free (i.e. 0 ppm of antimony).

The antimony content of the PET may be measured by microwave digestion of the PET resin under pressure ($HNO_3$ and HCL) and subsequent determination of antimony by Inductively coupled plasma mass spectrometry (ICP-MS). Determination of antimony content in PET can, for example, be done by GALAB Laboratories GmbH, Hamburg; Germany. Measuring the antimony content in the PET in accordance with ISO 105 E04, which uses less harsh digestion methods and Artificial Acid Sweat Solution, may not lead to determination of the complete antimony content in the PET, so this method should not be followed for the present invention. The a* value of the staple fibers may be less than -0.6, or less than -0.7, or from -2.0 to -0.6, or from -1.5 to -0.7, or from -1.5 to -0.8.

The b* value of the staple fibers may be higher than 1.5, or higher than 1.8, or from 1.5 to 5.0, or from 1.5 to 3.5.

The delta E* between the staple fibers alone (i.e. without latex binder) and the nonwoven web comprising the staple fibers and the latex binder, may be at least 1, or may be at least 2, or may be at least 3, or may be at least 4, or may be at least 5.

The delta E* between the staple fibers alone (i.e. without latex binder) and the nonwoven web comprising the staple fibers and the latex binder, may not be more than 10, or may not be more than 8, or may not be more than 7.5.

The latex binder has an opacity of at least 20%, or the latex binder may have an opacity of at least 25%, or at least 30%, or at least 35%, or at least 40%. The latex binder may have an opacity of less than 90%, or less than 80%. The nonwoven web comprising the staple fibers comprising PET having less than 150 ppm of antimony, may be covered by one or more first cover layer(s) towards the wearer-facing surface of the absorbent article, the one or more first cover layer(s) comprising or consisting of the topsheet, such that the nonwoven web does not form the wearer-facing surface of the absorbent article.

Alternatively, or in addition, the nonwoven web comprising the staple fibers comprising PET having less than 150 ppm of antimony, may be covered by one or more second cover layer(s) towards the garment-facing surface of the absorbent article, the one or more second cover layer(s) comprising or consisting of the backsheet, such that the nonwoven web does not form the garment-facing surface of the absorbent article.

The absorbent core of the absorbent article may comprise a combination of cellulose fibers and superabsorbent polymer particles, and the absorbent core may comprise areas which are free of cellulose fibers and superabsorbent polymer particles. The areas being free of cellulose fibers and superabsorbent polymer particles may be elongated areas having a length of from 20% and 80%, or from 20% to 70%, or from 30% to 60%, by total longitudinal dimension of the absorbent article.

BRIEF DESCRIPTION OF THE DRAWINGS

While the specification concludes with claims particularly pointing out and distinctly claiming the present invention, it is believed that the same will be better understood from the following description read in conjunction with the accompanying drawings in which:

Fig. 1 is an exemplary absorbent article in the form of a diaper.

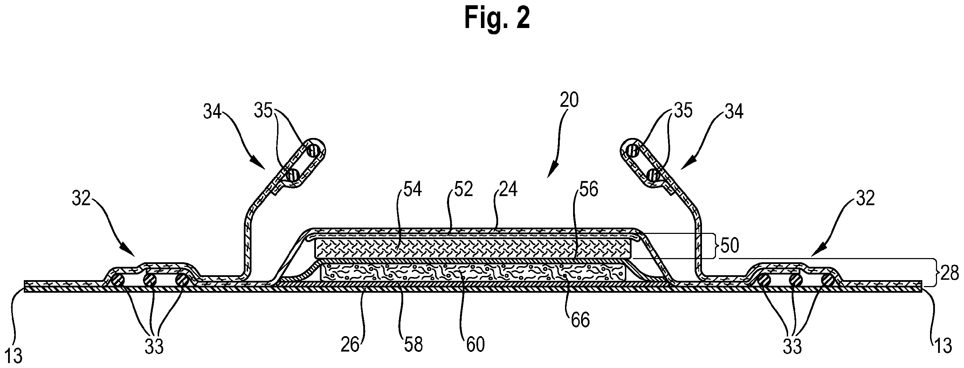

Fig. 2 is a transversal cross-section of the diaper of Fig. 1.

DETAILED DESCRIPTION OF THE INVENTION

**Definition of terms**

As used herein, "absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants (for babies or for adults), absorbent inserts (which are intended to be inserted into an outer cover to form a diaper or pant), feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable pants.

The term "absorbent core" as used herein refers to a component, which is placed or is intended to be placed within an absorbent article and which comprises an absorbent material enclosed in a core wrap. The term "absorbent core" does not include an acquisition or distribution layer or any other component of an absorbent article which is not either an integral part of the core wrap or placed within the core wrap. The absorbent core is typically the component of an absorbent article which comprises all, or at least the majority of, superabsorbent polymer and has the highest absorbent capacity of all the components of the absorbent article.

"Bicomponent" refers to fibers having a cross-section comprising two discrete polymer components, two discrete blends of polymer components, or one discrete polymer component and one discrete blend of polymer components. "Bicomponent fiber" is encompassed within the term "Multicomponent fiber." A bicomponent fiber may have an overall cross section divided into two subsections of the differing components of any shape or arrangement, including, for example, concentric core-and-sheath subsections, eccentric core-and-sheath subsections, side-by-side subsections, radial subsections, etc.

"Color", as used herein, includes any color in the CIELAB color space including primary color, secondary color, tertiary color, the combination thereof, as well as black and white.

CIE L*a*b* ("CIELAB") is the most commonly used color space specified by the International Commission on Illumination (French Commission internationale de l'éclairage, hence its CIE initialism). It describes all the colors visible to the human eye and was created to serve as a device independent model to be used as a reference.

The three coordinates of CIELAB represent the lightness of the color (L* = 0 yields black and L* = 100 indicates white), its position between red/magenta and green (a*, negative values indicate green while positive values indicate magenta) and its position between yellow and blue (b*, negative values indicate blue and positive values indicate yellow). The asterisk (*) after L, a and b are part of the full name, since they represent L*, a* and b*, to distinguish them from Hunter's L, a, and b.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essential of' which excludes any element, step or ingredient not mentioned which materially affect the way the feature performs its function, and the term "consisting of' which excludes any element, step, or ingredient not specified. Any preferred or exemplary embodiments described below are not limiting the scope of the claims, unless specifically indicated to do so.

As used herein, "diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be preformed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

As used herein, "disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use.

"Monocomponent" refers to fiber formed of a single polymer component or single blend of polymer components, as distinguished from bicomponent or multicomponent fiber.

"Multicomponent" refers to fiber having a cross-section comprising two or more discrete polymer components, two or more discrete blends of polymer components, or at least one discrete polymer component and at least one discrete blend of polymer components. "Multicomponent fiber" includes, but is not limited to, "bicomponent fiber." A multicomponent fiber may have an overall cross section divided into subsections of the differing components of any shape or arrangement, including, for example, coaxial subsections, concentric core-and-sheath subsections, eccentric core-and-sheath subsections, side-by-side subsections, islands-in the sea subsection, segmented pie subsections, etc.

A "nonwoven web" is a manufactured web of directionally or randomly oriented fibers, consolidated and bonded together, e.g. by one or more patterns of bonds and bond impressions created through localized compression and/or application of heat or ultrasonic energy, or a combination thereof. The fibers may, alternatively or in addition, be consolidated by the use of a binder. The binder may be provided in the form of binder fibers (which are subsequently molten) or may be provided in liquid, such as a styrene butadiene binder. A liquid binder is provided to the fibers (e.g. by spraying, printing or foam application) and is subsequently cured to solidify. The term "nonwoven" does not include fabrics which are woven, knitted, or stitch-bonded with yarns or filaments. The fibers may be of natural or man-made origin and may be staple or continuous filaments. Nonwoven fabrics can be formed by many processes such as meltblowing, spunlaid, solvent spinning, electrospinning, and carding. As used herein, "spunlaid" refers to fibers made by spunbond technology without having undergone further processing, such as bonding. The basis weight of nonwoven fabrics is usually expressed in grams per square meter ($g/m^2$). For the present invention, a multilayered nonwoven web may be consolidated and bonded by hydroentanglement and/or needle punching, in addition to being consolidated and bonded by bonds obtained by heat and/or compression (including ultrasonic bonding), e.g. in order to impart improved loft to the nonwoven web. Carded webs are formed of short, so-called staple fibers. They are typically formed into a layer of fibers and subsequently consolidated into a nonwoven web, for example by applying a binder to the fibers (as described above), by autogenously bonding the fibers together with heat and/or by intertwining the fibers by known processes such as hydroentangling or needle-punching. The carded fibers may also be bonded together, e.g. by one or more patterns of bonds and bond impressions created through localized compression and/or application of heat or ultrasonic energy, or a combination thereof.

As used herein, a "pantiliner" and a "sanitary napkin" generally have two end regions and a middle region (i.e. a crotch region). The pantiliner and the sanitary napkin have a body-facing surface and a garment facing surface. The size and shape of the absorbent structure positioned between the topsheet and the backsheet can be altered to meet absorbent capacity requirements, and to provide comfort to the wearer. The garment facing surface of the pantiliner and of the sanitary napkin can have thereon pressure sensitive adhesive for affixing to a wearer's undergarments. Typically, such adhesive is covered with a release strip which is removed before affixing to the undergarment. Pantiliners can also be provided with lateral extensions known commonly in the art as "flaps" or "wings" intended to extend over and cover the panty elastics in the crotch region of the user's undergarment. However, wings are normally not used with pantiliners but are more often used in sanitary napkins. Sanitary napkins and pantiliners of the present invention comprise barrier leg cuffs.

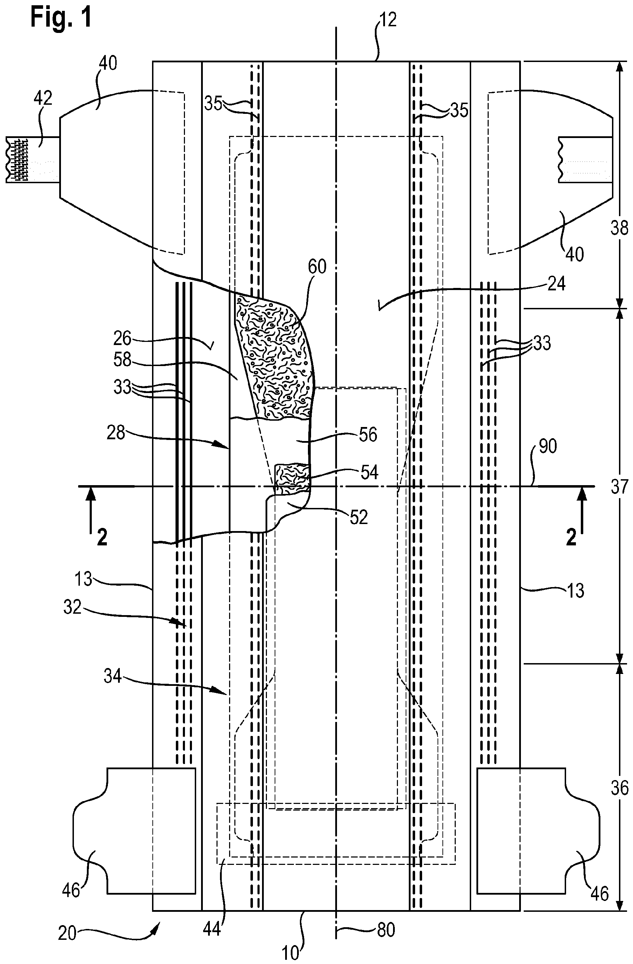

In more details, Figure 1 is a plan view of an exemplary diaper 20, in a flat-out state, with portions of the diaper being cut-away to more clearly show the construction of the diaper 20. As said, this diaper 20 is shown for illustration purpose only as the structure of the present invention may be comprised in a wide variety of diapers or other absorbent articles, such as pants.

As shown in Figure 1, the absorbent article, here a diaper, can comprise a liquid pervious topsheet 24, a liquid impervious backsheet 26, an absorbent core 28 which is positioned between the topsheet 24 and the backsheet 26. The absorbent core 28 can absorb and contain liquid received by the absorbent article and may comprise absorbent materials 60, such as superabsorbent polymers 66 and/or cellulose fibers, as well as other absorbent and non-absorbent materials commonly used in absorbent articles (e.g. thermoplastic adhesives immobilizing the superabsorbent polymer particles). The absorbent article of the present invention, such as the diaper 20 illustrated in Fig. 1, may optionally also include an acquisition system with an upper 52 and lower 54 acquisition layer.

The diaper also comprises barrier leg cuffs 34 and may further comprise elasticized leg cuffs 32. Moreover, the absorbent article may comprise a fastening system, such as an adhesive fastening system or a hook and loop fastening member, which can comprise tape tabs 42, such as adhesive tape tabs or tape tabs comprising hook elements, cooperating with a landing zone 44 (e.g. a nonwoven web providing loops in a hook and loop fastening system).

The diaper or pant, such as the diaper 20 shown in Figure 1 can be notionally divided in a first waist region 36, a second waist region 38 opposed to the first waist region 36 and a crotch region 37 located between the first waist region 36 and the second waist region 38. The longitudinal centerline 80 is the imaginary line separating the diaper along its length in two equal halves. The transversal centerline 90 is the imagery line perpendicular to the longitudinal line 80 in the plane of the flattened out diaper and going through the middle of the length of the diaper (the same applies to for the transversal centerline and longitudinal line of other absorbent articles of the present invention). The periphery of the diaper 20 is defined by the outer edges of the diaper 20. The longitudinal edges of the diaper may run generally parallel to the longitudinal centerline 80 of the diaper 20 and the end edges run between the longitudinal edges generally parallel to the transversal centerline 90 of the diaper 20. The crotch region, the first and the second waist region each constitutes 1/3 of the absorbent article along the longitudinal centerline.

Further, the diaper may comprise other elements, such as a back waist feature, which may be non-elastic or elastic, and a front waist feature, which may be non-elastic or elastic, a lotion applied onto the wearer-facing surface of the topsheet, back ears 40, and/or front ears 46.

The front and/or back ears 40, 46 may be separate components attached to the diaper or may instead be continuous with portions of the topsheet and/or backsheet -and/or even portions of the absorbent core - such that these portions form all or a part of the front and/or back ears 40, 46. Also combinations of the aforementioned are possible, such that the front and/or back ears 40, 46 are formed by portions of the topsheet and/or backsheet while additional materials are attached to form the overall front and/or back ears 40, 46. The front and/or back ears may be elastic or non-elastic.

The topsheet 24, the backsheet 26, and the absorbent core 28 may be assembled in a variety of well known configurations, in particular by gluing, heat embossing, ultrasonic bonding or combinations thereof. Exemplary diaper configurations are described generally in US3,860,003; US5,221,274; US5,554,145; US5,569,234; US5,580,411; and US6,004,306.

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polypropylene or bicomponent PE/PP fibers or mixtures thereof), or a combination of natural and synthetic fibers. The topsheet may have one or more layers. The topsheet may be apertured, may have any suitable three-dimensional features, and/or may have a plurality of embossments (e.g., a bond pattern). Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

The backsheet may comprise a backsheet outer cover material (sometimes referred to as a backsheet nonwoven) 40. The backsheet outer cover material may comprise one or more nonwoven materials joined to a backsheet film 28 and that covers the backsheet 28. The outer cover material 40 may form the garment-facing surface of the backsheet so that film is not present on the garment-facing surface. The backsheet outer cover material 40 may comprise a bond pattern, apertures, and/or three-dimensional features.

As set out in the background section above, PET resin which has been produced with a catalyst other than antimony generally leads to a resin with a yellowish color. Therefore, while the inventors found it desirable to use PET resin which has been produced with a catalyst other than antimony, they realized that the yellow color will not be accepted by consumers.

When considering the known processes described in the background section, which attempt to avoid the yellowing of the PET resin with other catalyst systems, it has been found that these processes typically apply substances such as inks, or phosphorous compounds. The presence of such substances in the PET resin has been identified as not being desirable, as they may give rise to health and environment related concerns as well, so replacing antimony with another substance that might be considered as problematic, has been found to be non-preferred.

The inventors have found that a nonwoven web comprising PET can be used in absorbent articles despite the yellowish color of the PET resin having less than 150 ppm of antimony, if the PET is comprised by staple fibers used in a carded nonwoven web which has been consolidated with a latex resin.

Staple fibers comprising PET resin made by a process not using antimony as a catalyst have been subjected to color tests, determining the a* and b* value as well as the L* value of these fibers. The same fibers have been color tested after a latex binder has been applied onto the staple fibers and latex binder has been cured to form a nonwoven web. It has been found that the latex binder forms a matrix in the nonwoven web which leads to a significant change of the a*, b* and L* values. I.e. the yellowish look of the PET fibers was no longer visible, or was only visible to a significantly lower extent.

The nonwoven web comprising staple fibers, wherein the staple fibers comprise PET with less than 150 ppm antimony, may be provided between the topsheet and the absorbent core. It may be in direct contact with the topsheet of the absorbent article.

For example, the absorbent article may comprise an acquisition system provided between the absorbent core and the topsheet and the nonwoven web, wherein the staple fibers comprise PET with less than 150 ppm antimony, may be comprised by the acquisition system, such as forming an upper layer of an acquisition system which is in direct contact with the topsheet.

Alternatively or in addition to being provided between the topsheet and the absorbent core, the nonwoven web, wherein the staple fibers comprise PET with less than 150 ppm antimony, may also be provided between the backsheet and the absorbent core of the absorbent article.

Still alternatively or in addition to being provided between the topsheet and the absorbent core, and/or between the backsheet and the absorbent core, the nonwoven web, wherein the staple fibers comprise PET with less than 150 ppm antimony, may be comprised by or may form the topsheet and/or the backsheet. In such circumstances, the nonwoven web may form that portion of the wearer-facing surface and/or the garment-facing surface of the absorbent article, which is formed by the topsheet and/or backsheet, respectively.

The PET having less than 150 ppm of antimony may also be free of dyes, pigments, hues and optical brighteners, as such compounds include substances which have recently also gained increased attention with regard to potential adverse effects for the human health and the environment. The same applies to phosphorous compounds which are comprised by some antimony-free PET resins previously suggested, where a phosphorus stabilizer is used in the PET resin manufacturing process to reduce the yellowish color. Also, some antimony-free PET resins described in the art may comprise trimellitic anhydride, and triethyl phosphonoacetate (TEPA) and the presence of these compounds is not desirable for use of the PET resin in absorbent articles.

The nonwoven web (as a whole), wherein the staple fibers comprise PET with less than 150 ppm antimony, may be free of dyes, pigments, hues and optical brighteners, and/or may be free of phosphorous substances, and/or may be free of trimellitic anhydride, and triethyl phosphonoacetate.

The nonwoven webs, wherein the staple fibers comprise PET with less than 150 ppm antimony, comprise at least 10%, by weight of the nonwoven web, of such PET. The nonwoven webs may comprise at least 30%, or at least 50%, or at least 70%, or 100%, by weight of the staple fibers, of PET having less than 150 ppm of antimony. If the nonwoven web comprises less than 100%, by weight of the staple fibers, of staple fibers comprising PET with less than 150 ppm of antimony, the nonwoven web may further comprise staple fibers formed of a thermoplastic material other than PET, such as polyolefin, polyamide, or specifically polypropylene (PP), polyethylene (PE), poly-lactic acid (PLA), Nylon 6-6 as well as combinations thereof (such as blends and copolymers).

The nonwoven web may not comprise any PET having more than 150 ppm, or more than 100 ppm, or more than 75 ppm, or more than 50 ppm, or more than 10 ppm of antimony, or may not comprise any PET which is not completely antimony-free (i.e. zero ppm).

The absorbent article may not comprise any PET having more than 150 ppm, or more than 100 ppm, or more than 75 ppm, or more than 50 ppm, or more than 10 ppm of antimony, or may not comprise any PET which is not completely antimony-free (i.e. zero ppm).

The PET having less than 150 ppm of antimony may be provided as homopolymer of PET, as copolymer (co-PET) or as a combination thereof. A combination thereof may include a mixture of fibers comprising homopolymer of PET and fibers comprising co-PET.

PET consists of polymerized units of the monomer ethylene terephthalate, with repeating ($C_{10}H_8O_4$) units.

In co-PET, for example, cyclohexane dimethanol (CHDM) can be added to the polymer backbone in place of ethylene glycol. Since this building block is much larger (6 additional carbon atoms) than the ethylene glycol unit it replaces, it does not fit in with the neighboring chains the way an ethylene glycol unit would. This interferes with crystallization and lowers the polymer's melting temperature. In general, such PET is known as PETG or PET-G (Polyethylene terephthalate glycol-modified; Eastman Chemical, SK Chemicals, and Artenius Italia are some PETG manufacturers).

Another common modifier for obtaining co-PET is isophthalic acid, replacing some of the 1,4-(*para*-) linked terephthalate units. The 1,2-(*ortho*-) or 1,3-(*meta*-) linkage produces an angle in the chain, which also disturbs crystallinity.

All of the staple fibers of the nonwoven web may be formed from thermoplastic material, such as polyolefin, polyamide or specifically polypropylene (PP), polyethylene (PE), poly-lactic acid (PLA), polyethylene terephthalate (PET), Nylon 6-6 as well as combinations thereof (such as blends and copolymers), in addition to comprising at least 10% by weight of the staple fibers comprising PET with less than 150 ppm (these 10% fibers are also formed from thermoplastic material). However, the nonwoven web may, in addition to the at least 10%, by weight of the staple fibers comprising PET with less than 150 ppm, also comprise fibers made of non-thermoplastic fibers, such as natural fibers. Such natural fibers include, for example, cotton or cellulose fibers. The natural fibers may be provide as one or more separate layers in the nonwoven web, and/or they be mixed with the other, non-natural, fibers.

Generally, resins including PP may be particularly useful because of polypropylene's relatively low cost, low density and surface friction properties of fibers formed from it (i.e., they have a relatively smooth, slippery tactile feel), as well as their good mechanical properties. Resins including PE may also be desirable because of polyethylene's relative softness/pliability and even more smooth/slippery surface friction properties. Relative each other, PP currently has a lower cost and fibers formed from it have a greater tensile strength, while PE currently has a greater cost and fibers formed from it have a lower tensile strength but greater pliability and a more smooth/slippery feel. Multicomponent fibers from PP and PE are desirable for as they combine the good softness properties of PP and the good mechanical properties of PE.

The thermoplastic polymer suitable for the staple fibers comprised by the nonwoven webs of the present invention may also be thermoplastic starch. As used herein, "thermoplastic starch" or "TPS" means a native starch or a starch derivative that has been rendered destructured and thermoplastic by treatment with one or more plasticizers, with at least one starch plasticizer still remaining. Thermoplastic starch compositions are well known and disclosed in several patents, for example: U.S. Patent Nos. 5,280,055; 5,314,934; 5,362,777; 5,844,023; 6,214,907; 6,242,102; 6,096,809; 6,218,321; 6,235,815; 6,235,816; and 6,231,970.

The nonwoven web may have any basis weight. However, relatively higher basis weight, while having relatively greater apparent caliper and loft, also has relatively greater cost.

The basis weight for the nonwoven webs comprising a latex binder and staple fibers with PET with less than 150 ppm of antimony, may be 200 $g/m^2$ or less, or may be from 5 $g/m^2$ to 120 $g/m^2$, or from 10 $g/m^2$ to 100 $g/m^2$, or from 15 $g/m^2$ to 80 $g/m^2$, or from 30 $g/m^2$ to less than 60 $g/m^2$.

It may generally be desirable to have nonwoven webs with relatively homogeneous distribution of the staple fibers and with relatively homogeneous distribution of the latex binder, especially for nonwoven webs with relatively low basis weight. If the nonwoven web comprises less than 100% of staple fibers comprising PET with less than 150 ppm of antimony, the staple fibers comprising such PET may also be distributed homogeneously within the nonwoven web.

The carded nonwoven web, comprising staple PET with less than 150 ppm of antimony, also comprises a latex binder, which has been cured after application onto the fibers to solidify.

For example, the nonwoven web comprising staple fibers, comprising PET with less than 150 ppm of antimony, may comprise at least 50%, or from 50% to 90%, or from 60% to 80%, by weight of the nonwoven web, of staple fibers, and at least 10%, or from 10% to 50%, or from 20% to 40%, by weight of the carded nonwoven web, of a latex binder.

The nonwoven web may comprise at least 30%, or at least 50%, or at least 70%, or 100%, by weight of the staple fibers, of PET with less than 150 ppm of antimony. All staple fibers of the nonwoven web may comprise or consist of PET with less than 150 ppm of antimony.

Staple fibers are short fibers. They may have a length of from 10 mm to 120 mm, or from 25 mm to 80 mm, or from 25 mm to 60 mm. The staple fibers may be straight or, alternatively, may have two-dimensional or three-dimensional crimp. Crimped staple fibers can improve the resiliency of the nonwoven web, which is generally desirable when the nonwoven web is comprised by an acquisition system of the absorbent article.

A mechanical process when using staple fibers, is carding. To obtain staple fibers, the fibers are first spun, cut to a few centimeters length. The cut fibers may be put into bales (bundles of compressed fibers). The carding process may then start with the opening of the bales of fibers which may be blended and are typically conveyed to the next stage by air transport. They are subsequently combed into a fiber compound (typically a layer of staple fibers) by a carding machine, such as a rotating drum or series of drums covered in fine wires or teeth. The precise configuration of cards will depend for example, on the basis weight and fiber orientation required. The web can be parallel-laid, where most of the fibers are laid in the direction of the web travel, or they can be random-laid.

The staple fibers comprising PET with less than 150 ppm of antimony, used in the nonwoven web, may be monocomponent fibers, as multicomponent fibers, or combinations thereof. Suitable multicomponent fibers are bicomponent fibers, such as core/sheath bicomponent fibers and side-by-side bicomponent fibers. The core/sheath bicomponent fibers may be concentric or eccentric fibers.

The shape of the staple fibers of the nonwoven web, comprising staple fibers with PET having less than 150 ppm of antimony, may be round (i.e. fibers having a circular cross-section). Alternatively, the staple fibers may have non-round shape, such as multilobal fibers (e.g. trilobal fibers), flat fibers ("ribbon-like" cross-section), rhomboid fibers or triangular fibers. In multilobal fibers, a central section is encircled by a multiplicity of lobes. E.g. in a trilobal fiber, the central section is encircled by three lobes. The nonwoven web may also comprise a mixture of staple fibers having different shapes, such as a mixture of round and multilobal fibers.

The staple fibers of the nonwoven web, comprising staple fibers with PET having less than 150 ppm of antimony, may be solid or hollow. Alternatively, a mixture of solid and hollow staple fibers can be used. The solid fibers may or may not have a different shape than the hollow fibers.

## Latex binder

The nonwoven web comprising staple fibers, comprising PET with less than 150 ppm of antimony, is a carded nonwoven web and comprises at least 10%, by weight of the carded nonwoven web, of a latex binder. The carded nonwoven web may comprise at least 10%, or from 10% to 50%, or from 20% to 40%, by weight of the carded nonwoven web, of a latex binder.

A suitable latex binder can be prepared by a process including the steps of:

(1) polymerizing a monomer mixture comprising styrene, itaconic acid, surfactant and water soluble free radical initiator to form a seed;

(2) sequentially adding equal increments of a monomer mixture of styrene, butadiene and acrylic acid to the seed under emulsion polymerization conditions to form a styrene-butadiene-acrylic acid copolymer; and then

(3) neutralizing the styrene-butadiene-acrylic acid copolymer to a pH of about 4.5 to 7 to form the latex binder.

The binder is applied onto the carded nonwoven material (which will typically not yet be a "stable" web in the sense of a consolidated material). Subsequently, the latex binder is cured, using methods well known in the art, such as by application of heat or radiation. The term "cured" refers to the latex binder being cross-linked. The curing of the treated staple fibers is affected by a temperature above the glass transition temperature of the binder.

The latex binder may be prepared by well-known conventional emulsion polymerization techniques using one or more ethylenically unsaturated monomers and a polymeric surfactant as herein disclosed and additional conventional additives such as free-radical initiators, optional chain transfer agents, chelating agents and the like can be utilized as set forth in U.S. Pat. No. 5,166,259 to Schmeing and White.

In accordance with a preferred embodiment, the latex is prepared by polymerizing a monomer mixture comprising styrene, itaconic acid, surfactant and a water soluble free radical initiator to form a seed. A monomer mixture is then added incrementally to the seed under emulsion polymerization conditions. The monomer mixture includes styrene, butadiene, and acrylic acid. The acrylic acid can help in the cross-linking process of the binder upon curing. The monomer mixture is preferably added incrementally to the seed to form a styrene-butadiene-acrylic acid copolymer. In a preferred embodiment, the monomer mixture includes about 34-70 weight % styrene of the total composition. The monomer mixture also includes about 0.5-2.5 weight % itaconic acid, preferably 2 weight % itaconic acid of the total composition, about 20-55 weigt % butadiene and acrylic acid in an amount of about 6-10 weight %, preferably 8 weight %.

A surfactant is added to the monomer mixture in an amount of about 0.05-2.0 weight %. The surfactant may be most any suitable emulsifier, soap, or the like well known in the art and suitable at the pH of the latex. Examples of suitable emulsifiers and surfactants include alkyl sulfates, alkyl sulfosuccinates, alkyl aryl sulfonates, alpha-olefin sulfonates, fatty or rosin acid salts, only or octyl phenol reaction products of ethylene oxide and the like. Other surfactants that may be used include those identified in Surface Active Agents, Schwartz and Berry, Vol. 1, Interscience Publishers, Inc., New York, 1958; Surface Activity, Moilet, Collie and Black, D. Van Nostrand Company, Inc., New York, 1961; Organic Chemistry, Feiser and Feiser, D.C. Heath and Company, Boston, 1944; and The Merck Index, Seventh Edition, Merck & Co., Inc., Rahway, N.J., 1960, all of which are hereby incorporated by reference.

The copolymer is then neutralized to a pH of about 4.5 to 7.0 to form the latex. The pH of the latex is neutralized by addition of a base. Examples of a suitable base include potassium hydroxide, sodium bicarbonate, ammonium hydroxide, sodium hydroxide and the like. The amount of base added to the latex is adjusted to obtain the desired pH range as is well known in the art.

Polymerization is typically carried out from about 65°C to 75°C. Polymerization is generally conducted for about 4 to 24 hours, however polymerization conditions may vary as desired to provide different conversion levels of monomer to copolymer. The monomer mixture is allowed to react until substantially constant solids at which time at least 99% of the monomers have been converted.

The liquid binder is provided to the fibers e.g. by spraying, printing or foam application and is subsequently cured to solidify. The liquid binder may be provided onto the staple fibers such that the solidified latex binder is homogenously distributed. "Homogeneously distributed liquid binder" as used herein means, that the amount of liquid binder per 0.01 m$^2$ sample (10 cm by 10 cm sample randomly taken at 10 different locations of the nonwoven web comprising the staple fibers and latex binder) does not vary by more than 20%, or not more than 15%, or not more than 10%, or not more than 5% from the average amount of latex binder in the nonwoven web.

The latex binder may also comprise from 0.05% to 2.5%, or from 0.05% to 2.0%, or from 0.05% to 1.5%, or from 0.1% to 1%, by weight of the latex binder, of titanium dioxide. The use of titanium dioxide can help to increase the opacity of the latex binder.

### Test Methods

### Measurement of a*, b*, L* and delta E* values

The measurement is based on the CIE L* a* b* color system (CIELAB). L*, a* and b* may be measured using a 0.deg. illumination / 45.deg. detection, circumferential optical geometry, spectrophotometer with a computer interface such as the HunterLab LabScan® XE running Universal Software (available from Hunter Associates Laboratory Inc., Reston, VA). Instrument calibration and measurements are to be made using the standard protocol by the vendor. The HunterLab LabScan® XE is equipped with a Port Down Stand, which enables measurement of the sample from a straight-down angle. All testing is performed in a room maintained at about 23°C $\pm$ 2°C and about 50% $\pm$ 2% relative humidity.

Configure the spectrophotometer for the L*, a*, b* color value scale, D65 illuminant, 10.deg. standard observer, with UV filter set to nominal. Standardize the instrument according to the manufacturer's procedures using the 1.20 inch port size and 1.00 inch area view.

The specimen comprises all the staple fibers to be comprised by the nonwoven web, comprising all staple fibers of the nonwoven web (i.e. also those which do not comprise PET having less than 150 ppm of antimony), but not the latex binder. The staple fibers in the specimen have the same basis weight and composition (in case a mixture of different staple fibers is used) as the staple fibers in the nonwoven web to be used in the absorbent article. The L*, a*, b* values of the specimen are measured.

Then, a specimen of the nonwoven web comprising the staple fibers and the latex binder (i.e. a sample of the "final" nonwoven web as is to be comprised by the absorbent article) is prepared. To obtain the specimen, eight pieces of the nonwoven web, each being at least 31 mm by 31 mm, are cut and stacked up one on top of the other. The eight layer specimen is measured for L*, a*, b* values. The specimen needs to have a sample size of at least 31 mm by 31 mm.

The delta E* value is calculated.

Precondition all samples at about 23°C $\pm$ 2°C and about 50% $\pm$ 2% relative humidity for 2 hours prior to testing.

Place the specimen on the spectrophotometer. The specimen should completely cover the port.

A total of three substantially identical samples are analyzed and their L*, a*, b* results recorded. Calculate and report the average values and standard deviation for the staple fibers / nonwoven web with staple fibers and latex binder measurements to the nearest 0.01%.

Record the averaged values as $L^*_1$, $a^*_1$ and $b^*_1$ for the specimen with the staple fibers but not comprising the latex binder, and the averaged values as $L^*_2$, $a^*_2$ and $b^*_2$ for the specimen comprising the staple fibers and the latex binder. Calculate and report the color difference (delta E*) between the staple fibers taken alone, and the nonwoven web including the latex binder, using the following equation:

$$\text{delta } E^* = \sqrt{(L^*_2 - L^*_1)^2 + (a^*_2 - a^*_1)^2 + (b^*_2 - b^*_1)^2}$$

### Opacity Measurement Method

The opacity of a material is the degree to which light is blocked by that material. A higher opacity value indicates a higher degree of light block by the material. Opacity may be measured using a 0.deg. illumination / 45.deg. detection, circumferential optical geometry, spectrophotometer with a computer interface such as the HunterLab LabScan® XE running Universal Software (available from Hunter Associates Laboratory Inc., Reston, VA). Instrument calibration and measurements are made using the standard white and black calibration plates provided by the vendor. All testing is performed in a room maintained at about 23°C $\pm$ 2°C and about 50% $\pm$ 2% relative humidity. Configure the spectrophotometer for the XYZ color scale, D65 illuminant, 10.deg. standard observer, with UV filter set to nominal. Standardize the instrument according to the manufacturer's procedures using the 1.20 inch port size and 1.00 inch area view. After calibration, set the software to the Y opacity procedure.

To obtain the specimen, the liquid latex binder is taken and formed into a film. The content of solid and the content of liquid (such as water) is noted. The amount of liquid latex binder used to prepare the specimen depends on the percentage of water in the liquid latex binder. For example, if the liquid binder comprises 80% of water and 20% solids, 5 g of liquid binder are used.

The 5 g of liquid binder having 20% of solids, is poured onto a Petri dish having a size of 100 mm by 100 mm (the height is not critical but may be 20 mm) to form a homogeneous layer. If the liquid binder has a solid content other than 20%, the amount of liquid binder poured into the Petri dish has to be adapted accordingly to obtain a film of 100 g/m$^2$ from the solid content in the liquid latex binder. The Petri dish is put in an oven at 150°C for at least 30 minutes until the water is evaporated and a film has been formed. The film is carefully removed from the Petri dishand measured for reference L*, a*, b* values.

Place the specimen over the measurement port. If it is too large, a piece of suitable size can be cut. The specimen should completely cover the port, i.e. the sample has to be at least 31 mm by 31 mm. Cover the specimen with the white standard plate. Take a reading, then remove the white tile and replace it with black standard tile without moving the specimen. Obtain a second reading, and calculate the opacity as follows:

$$\text{Opacity} = [\text{Y value (black backing)} / \text{Y value (white backing)}] \times 100\%$$

A total of five substantially identical samples are analyzed and their opacity results recorded. Calculate and report the average opacity and standard deviation for the film measurements to the nearest 0.01%.

## Claims

1. Absorbent article comprising a topsheet forming a wearer-facing surface of the absorbent article, a backsheet forming a garment-facing surface of the absorbent article and an absorbent core interposed between the topsheet and the backsheet,
   wherein the absorbent article comprises a nonwoven web, the nonwoven web being a carded nonwoven web,
   the nonwoven web comprises at least 50% by weight of the nonwoven web, of staple fibers and at least 10%, by weight of the nonwoven web, of a latex binder, wherein the latex binder has an opacity of at least 20%, as measured according to the test method set out herein;
   the staple fibers have an a* value of unequal zero and a b* value of unequal zero, as measured according to the test method set out herein, and comprise at least 10 %, by weight of the staple fibers, of polyethylene terephthalate (PET), the PET comprising less than 150 ppm of antimony, as measured according to the test method set out herein in the Summary of the Invention.

2. The absorbent article of claim 1, wherein delta E* value between the staple fibers alone and the nonwoven web comprising the staple fibers and the latex binder, is at least 1.0, or at least 2.0 or at least 3.0, as measured according to the test method set out herein.

3. The absorbent article of claim 1 or 2, wherein the a* value of the staple fibers is less than -0.6, as measured according to the test method set out herein.

4. The absorbent article of any of the preceding claims, wherein the b* value of the staple fibers is higher than 1.5, as measured according to the test method set out herein.

5. The absorbent article of any of the preceding claims, wherein the latex binder has an opacity of at least 25%.

6. The absorbent article of any of the preceding claims, wherein the PET comprised by the staple fibers has less than 100 ppm, or less than 50 ppm, or less than 75 ppm, or less than 10 ppm, or zero ppm of antimony.

7. The absorbent article of any of the preceding claims, wherein the nonwoven web is covered by one or more first cover layer(s) towards the wearer-facing surface of the absorbent article, the one or more first cover layer(s) comprising or consisting of the topsheet, such that the nonwoven web does not form the wearer-facing surface of the absorbent article.

8. The absorbent article of any of the preceding claims, wherein the nonwoven web is covered by one or more second cover layer(s) towards the garment-facing surface of the absorbent article, the one or more second cover layer(s) comprising or consisting of the backsheet, such that the nonwoven web does not form the garment-facing surface of the absorbent article.

9. The absorbent article of any of the preceding claims, wherein the nonwoven web is provided between the topsheet and the absorbent core.

10. The absorbent article of claim 9, wherein the absorbent article comprises an acquisition layer which is provided between the absorbent core and the topsheet, and wherein the nonwoven web is comprised by the acquisition system.

11. The absorbent article of any of the preceding claims, wherein the PET is a provided as homopolymer, copolymer (co-PET), or a combination thereof.

12. The absorbent article of any of the preceding claims, wherein the PET does not comprise any of the following: dyes, pigments, hues and optical brighteners.

13. The absorbent article of any of the preceding claims, wherein the PET does not comprise a phosphorous compound.

14. The absorbent article of any of the preceding claims, wherein the staple fibers are homogeneously distributed throughout the nonwoven web.

15. The absorbent article of any of the preceding claims, wherein the staple fibers comprising less than 150 ppm of antimony are homogeneously distributed throughout the nonwoven web.

16. The absorbent article of any of the preceding claims, wherein the latex binder is homogeneously distributed throughout the nonwoven web.

17. The absorbent article of any of the preceding claims, wherein the absorbent core comprises a combination of cellulose fibers and superabsorbent polymer particles, and the absorbent core comprises areas which are free of cellulose fibers and superabsorbent polymer particles, wherein the areas are elongated areas having a length of from 20% and 80% by total longitudinal dimension of the absorbent article.

**Patentansprüche**

1. Absorptionsartikel, umfassend eine Oberschicht, die eine trägerseitige Oberfläche des Absorptionsartikels bildet, eine Unterschicht, die eine bekleidungsseitige Oberfläche des Absorptionsartikels bildet, und einen Absorptionskern zwischen der Oberschicht und der Unterschicht,
wobei der Absorptionsartikel eine Vliesbahn umfasst, wobei die Vliesbahn eine kardierte Vliesbahn ist,
wobei die Vliesbahn zu mindestens 50 Gew.-% bezogen auf das Gewicht der Vliesbahn Stapelfasern und zu mindestens 10 Gew.-% bezogen auf das Gewicht der Vliesbahn ein Latexbindemittel umfasst, wobei das Latexbindemittel eine Trübung von mindestens 20 % aufweist, gemessen gemäß dem hierin dargelegten Prüfverfahren;
wobei die Stapelfasern einen a*-Wert von ungleich null und einen b*-Wert von ungleich null aufweisen, gemessen gemäß dem hierin dargelegten Prüfverfahren, und zu mindestens 10 Gew.-%, bezogen auf das Gewicht der Stapelfasern, Polyethylenterephthalat (PET) umfassen, wobei das PET weniger als 150 ppm Antimon umfasst, gemessen gemäß dem in der Kurzdarstellung der Erfindung dargelegten Prüfverfahren.

2. Absorptionsartikel nach Anspruch 1, wobei der delta E*-Wert zwischen den Stapelfasern allein und der Vliesbahn, die die Stapelfasern und das Latexbindemittel umfasst, mindestens 1,0 oder mindestens 2,0 oder mindestens 3,0 beträgt, gemessen gemäß dem hierin dargelegten Prüfverfahren.

3. Absorptionsartikel nach Anspruch 1 oder 2, wobei der a*-Wert der Stapelfasern kleiner als -0,6 ist, gemessen gemäß dem hierin dargelegten Prüfverfahren.

4. Absorptionsartikel nach einem der vorstehenden Ansprüche, wobei der b*-Wert der Stapelfasern höher als 1,5 ist, gemessen gemäß dem hierin dargelegten Prüfverfahren.

5. Absorptionsartikel nach einem der vorstehenden Ansprüche, wobei das Latexbindemittel eine Trübung von mindestens 25 % aufweist.

6. Absorptionsartikel nach einem der vorstehenden Ansprüche, wobei das PET, das die Stapelfasern umfasst, weniger als 100 ppm oder weniger als 50 ppm oder weniger als 75 ppm oder weniger als 10 ppm oder null ppm Antimon aufweist.

7. Absorptionsartikel nach einem der vorstehenden Ansprüche, wobei die Vliesbahn durch eine oder mehrere erste Deckschicht(en) in Richtung der trägerseitigen Oberfläche des Absorptionsartikels bedeckt ist, wobei die eine oder die mehreren ersten Deckschicht(en) die Oberschicht umfassen oder daraus bestehen, derart, dass die Vliesbahn nicht die trägerseitige Oberfläche des Absorptionsartikels bildet.

8. Absorptionsartikel nach einem der vorstehenden Ansprüche, wobei die Vliesbahn durch eine oder mehrere zweite Deckschicht(en) in Richtung der bekleidungsseitigen Oberfläche des Absorptionsartikels bedeckt ist, wobei die eine oder die mehreren zweiten Deckschicht(en) die Unterschicht umfassen oder daraus bestehen, derart, dass die Vliesbahn nicht die bekleidungsseitige Oberfläche des Absorptionsartikels bildet.

9. Absorptionsartikel nach einem der vorstehenden Ansprüche, wobei die Vliesbahn zwischen der Oberschicht und dem Absorptionskern vorgesehen ist.

10. Absorptionsartikel nach Anspruch 9, wobei der Absorptionsartikel eine Aufnahmeschicht umfasst, die zwischen dem Absorptionskern und der Oberschicht vorgesehen ist, und wobei die Vliesbahn durch das Aufnahmesystem gebildet ist.

11. Absorptionsartikel nach einem der vorstehenden Ansprüche, wobei das PET ein als Homopolymer, Copolymer (Co-PET) oder eine Kombination davon bereitgestellt ist.

12. Absorptionsartikel nach einem der vorstehenden Ansprüche, wobei das PET nichts der Folgenden umfasst: Farbstoffe, Pigmente, Tönungsmittel und optische Aufheller.

13. Absorptionsartikel nach einem der vorstehenden Ansprüche, wobei das PET keine Phosphorverbindung umfasst.

14. Absorptionsartikel nach einem der vorstehenden Ansprüche, wobei die Stapelfasern homogen in der Vliesbahn verteilt sind.

15. Absorptionsartikel nach einem der vorstehenden Ansprüche, wobei die Stapelfasern, die weniger als 150 ppm Antimon umfassen, homogen in der Vliesbahn verteilt sind.

16. Absorptionsartikel nach einem der vorstehenden Ansprüche, wobei das Latexbindemittel homogen in der Vliesbahn verteilt ist.

17. Absorptionsartikel nach einem der vorstehenden Ansprüche, wobei der Absorptionskern eine Kombination von Cellulosefasern und Superabsorberpolymerteilchen umfasst und der Absorptionskern Bereiche umfasst, die frei von Cellulosefasern und Superabsorberpolymerteilchen ist, wobei die Bereiche langgestreckte Bereiche mit einer Länge von 20 % und 80 % nach Gesamtlängsabmessung des Absorptionsartikels aufweisen.

## Revendications

1. Article absorbant comprenant une feuille de dessus formant une surface faisant face au porteur de l'article absorbant, une feuille de fond formant une surface faisant face au vêtement de l'article absorbant et une âme absorbante intercalée entre la feuille de dessus et la feuille de fond,
dans lequel l'article absorbant comprend une nappe non tissée, la nappe non tissée étant une nappe non tissée cardée,
la nappe non tissée comprend au moins 50 % en poids de la nappe non tissée, de fibres discontinues et au moins 10 %, en poids de la nappe non tissée, d'un liant à base de latex, dans lequel le liant à base de latex a une opacité d'au moins 20 %, telle que mesurée selon le procédé de test présenté ici ;
les fibres discontinues ont une valeur a* non égale à zéro et une valeur b* non égale à zéro, telles que mesurées selon le procédé de test présenté ici, et comprennent au moins 10 %, en poids des fibres discontinues, de téréphtalate de polyéthylène (PET), le PET comprenant moins de 150 ppm d'antimoine, tel que mesuré selon le procédé de test présenté ici dans le Résumé de l'invention.

2. Article absorbant selon la revendication 1, dans lequel une valeur delta E* entre les fibres discontinues seules et la nappe non tissée comprenant les fibres discontinues et le liant à base de latex, vaut au moins 1,0, ou au moins 2,0 ou au moins 3,0, telle que mesurée selon le procédé de test présenté ici.

3. Article absorbant selon la revendication 1 ou 2, dans lequel la valeur a* des fibres discontinues est inférieure à -0,6, telle que mesurée selon le procédé de test présenté ici.

4. Article absorbant selon l'une quelconque des revendications précédentes, dans lequel la valeur b* des fibres discontinues est supérieure à 1,5, telle que mesurée selon le procédé de test présenté ici.

5. Article absorbant selon l'une quelconque des revendications précédentes, dans lequel le liant à base de latex a une opacité d'au moins 25 %.

6. Article absorbant selon l'une quelconque des revendications précédentes, dans lequel le PET compris dans les fibres discontinues a moins de 100 ppm, ou moins de 50 ppm, ou moins de 75 ppm, ou moins de 10 ppm, ou zéro ppm d'antimoine.

7. Article absorbant selon l'une quelconque des revendications précédentes, dans lequel la nappe non tissée est couverte par une ou plusieurs première(s) couche(s) de couverture en direction de la surface faisant face au porteur de l'article absorbant, la ou les première(s) couche(s) de couverture comprenant ou constituant la feuille de dessus, de telle sorte que la nappe non tissée ne forme pas la surface faisant face au porteur de l'article absorbant.

8. Article absorbant selon l'une quelconque des revendications précédentes, dans lequel la nappe non tissée est couverte par une ou plusieurs deuxième(s) couche(s) de couverture en direction de la surface faisant face au vêtement de l'article absorbant, la ou les deuxième(s) couche(s) de couverture comprenant ou constituant la feuille de fond, de telle sorte que la nappe non tissée ne forme pas la surface faisant face au vêtement de l'article absorbant.

9. Article absorbant selon l'une quelconque des revendications précédentes, dans lequel la nappe non tissée est fournie entre la feuille de dessus et l'âme absorbante.

10. Article absorbant selon la revendication 9, dans lequel l'article absorbant comprend une couche de recueil qui est fournie entre l'âme absorbante et la feuille de dessus, et dans lequel la nappe non tissée est comprise dans le système de recueil.

11. Article absorbant selon l'une quelconque des revendications précédentes, dans lequel le PET est fourni en tant qu'homopolymère, copolymère (co-PET), ou une combinaison de ceux-ci.

12. Article absorbant selon l'une quelconque des revendications précédentes, dans lequel le PET ne comprend pas l'un quelconque des suivants : des colorants, des pigments, des teintures et des azurants optiques.

13. Article absorbant selon l'une quelconque des revendications précédentes, dans lequel le PET ne comprend pas de composé phosphoreux.

14. Article absorbant selon l'une quelconque des revendications précédentes, dans lequel les fibres discontinues sont réparties de façon homogène sur l'ensemble de la nappe non tissée.

15. Article absorbant selon l'une quelconque des revendications précédentes, dans lequel les fibres discontinues comprenant moins de 150 ppm d'antimoine sont réparties de façon homogène sur l'ensemble de la nappe non tissée.

16. Article absorbant selon l'une quelconque des revendications précédentes, dans lequel le liant à base de latex est réparti de façon homogène sur l'ensemble de la nappe non tissée.

17. Article absorbant selon l'une quelconque des revendications précédentes, dans lequel l'âme absorbante comprend une combinaison de fibres de cellulose et de particules de polymère superabsorbant, et l'âme absorbante comprend des zones qui sont exemptes de fibres de cellulose et de particules de polymère superabsorbant, dans lequel les zones sont des zones allongées ayant une longueur allant de 20 % et 80 % par rapport à la dimension longitudinale totale de l'article absorbant.

# Fig. 2

EP 3 466 389 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 02420958
- EP 1316585 B1
- EP 1491572 A1
- EP 1153953 B1
- EP 1327648 A1
- US 20060014920 A1
- EP 1584723 A1
- EP 3216433 A1
- WO 9118036 A
- US 3860003 A
- US 5221274 A
- US 5554145 A
- US 5569234 A
- US 5580411 A
- US 6004306 A
- US 5280055 A
- US 5314934 A
- US 5362777 A
- US 5844023 A
- US 6214907 B
- US 6242102 B
- US 6096809 A
- US 6218321 B
- US 6235815 B
- US 6235816 B
- US 6231970 B
- US 5166259 A

**Non-patent literature cited in the description**

- **SCHWARTZ ; BERRY.** Surface Active Agents. Interscience Publishers, Inc, 1958, vol. 1
- **COLLIE ; BLACK, D.** Surface Activity, Moilet. Van Nostrand Company, Inc, 1961
- **FEISER ; FEISER, D.C.** Organic Chemistry. Heath and Company, 1944
- The Merck Index. Merck & Co., Inc, 1960